# EUROPEAN PATENT SPECIFICATION

(11) **EP 2 349 371 B1**
(45) Date of publication and mention of the grant of the patent: **04.12.2013**
(21) Application number: 09793310.5
(22) Date of filing: 06.10.2009
(51) Int. Cl.: A61L 29/08, A61L 29/16, A61L 27/34, A61L 27/54

(54) **MEDICAL DEVICES FOR DELIVERY OF THERAPEUTIC AGENTS TO BODY LUMENS**
MEDIZINISCHE VORRICHTUNGEN ZUR FREISETZUNG VON WIRKSTOFFEN IN KÖRPERLUMEN
DISPOSITIFS MÉDICAUX POUR ADMINISTRATION D'AGENTS THÉRAPEUTIQUES À DES LUMIÈRES CORPORELLES

(30) Priority: 07.10.2008 US 103290 P; 17.03.2009 US 160920 P
(43) Date of publication of application: 03.08.2011
(73) Proprietor: Boston Scientific Scimed, Inc., Maple Grove, MN 55311-1566 (US)
(72) Inventor: WEBER, Jan, NL-6228 GJ Maastricht (NL); SCHEUERMANN, Torsten, 80803 Munich (DE)
(74) Representative: Vossius & Partner
(86) International application number: PCT/US2009/059630
(87) International publication number: WO 2010/042471

(56) References cited:
- WO-A1-2005/115496
- WO-A2-2008/036357
- WO-A2-2010/019645
- WO-A2-2010/027678
- US-A1- 2005 129 727
- US-B1- 6 506 405

## Description

### FIELD OF THE INVENTION

The present invention is directed to medical devices for delivery of therapeutic agents to body lumens.

### BACKGROUND OF THE INVENTION

The in-situ delivery of therapeutic agents within the body of a patient is common in the practice of modem medicine. In-situ delivery of therapeutic agents is often implemented using medical devices that may be temporarily or permanently placed at a target site within the body. These medical devices can be maintained, as required, at their target sites for short or prolonged periods of time, in order to deliver therapeutic agents to the target site.

For example, in recent years, drug eluting coronary stents, which are commercially available from Boston Scientific Corp. (TAXUS, PROMUS), Johnson & Johnson (CYPHER) and others, have been widely used for maintaining vessel patency after balloon angioplasty. These products are based on metallic expandable stents with polymer coatings that release anti-restenotic drugs at a controlled rate and total dose.

WO2005/115496 discloses a medical device such as a balloon comprising a multilayer region that comprises a charged nanoparticle layer comprising charged nanoparticles, paclitaxel and albumin, and a plurality of charged polyelectrolyte layers comprising charged polyelectrolyte species.

US2005/129727 discloses nanocapsules bonded to the surface of medical devices comprising a drug-containing core and a polyelectrolyte multiplayer encapsulating the drug-containing core. The drug is include paclitaxel and the polyelectrolytes include albumin. WO2008/036357 discloses a medical device such as a balloon comprising protein-bound paclitaxel particles such as albumin-bound paclitaxel nanoparticles, e.g. Abraxane.

WO2010/019645 discloses an implantable or insertable medical device such as a balloon that comprises a conductive substrate and a cathodically electrodeposited coating over the substrate, said electrodeposited coating comprising a conductive polymer and a cationic polyelectrolyte.

WO 2010/027678 discloses a medical device comprising a substrate, at least one therapeutic agent disposed over or in the substrate, and at least one inorganic layer disposed over the therapeutic agent and the substrate, wherein the inorganic layer is either a porous inorganic layer or is a non-porous layer that becomes a porous inorganic layer in vivo. Specific therapeutic agents include, e.g., taxanes such as paclitaxel including particulate forms thereof, for instance, protein-bound paclitaxel particles such as albumin-bound paclitaxel nanoparticles, e.g., ABRAXANE.

Therapeutic agents have also been delivered to vessel walls using balloons. For example, recent clinical trials have shown that in-stent restenosis can be treated using a balloon having a sprayed coating of a mixture of paclitaxel and iopromide. B. Scheller et al., Eurointervention Supplement (2008) Vol. 4 (Supplement C) C63-C66.

### SUMMARY OF THE INVENTION

In accordance with one aspect, the invention provides a drug delivery device for delivery of a drug to the body. The device is provided with at least one layer that comprises drug-containing particles.

In certain embodiments, the drug delivery device comprises a radially expandable member and a layer of particles comprising a drug that are electrostatically attached to the surface of the radially expandable member.

In certain embodiments, the drug delivery device comprises a radially expandable member and a hard, brittle layer disposed over the radially expandable member that comprises (a) a binding material and (b) particles comprising a drug.

In certain embodiments, the drug delivery device comprises a radially expandable member, a hard, brittle layer disposed over the radially expandable member that comprises a binding material, and a layer disposed over the hard, brittle layer comprising a drug.

In accordance with another aspect, the invention provides methods of treatment using such devices.

### BRIEF DESCRIPTION OF THE DRAWINGS

Fig. 1 is a schematic illustration of a drug-containing particle in accordance with the prior art.

Fig. 2A is a schematic illustration of a balloon catheter in accordance with the present invention.

Fig. 2B is a schematic cross-section taken along line b---b of Fig. 2A.

Fig. 3A is a schematic illustration of portion of a drug delivery device in accordance with the present invention.

Fig. 3B is a schematic illustration of the drug delivery device portion of Fig. 3A after coming into contact with a cell wall.

### DETAILED DESCRIPTION OF THE INVENTION

In accordance with one aspect, the invention provides a drug delivery device for delivery of a drug to the body. The device is provided with at least one layer that comprises drug-containing particles. "Drugs," "therapeutic agents," "pharmaceuticals," "pharmaceutically active agents", and other related terms may be used interchangeably herein.

Particularly preferred are expandable devices which are configured such that they can be inserted into a body lumen (e.g., a blood vessel) and which can be radially expanded in vivo to contact the wall of the body lumen. Examples of such expandable devices include stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts, etc.), catheters (*e.g*., urological catheters or vascular catheters such as balloon catheters and various central venous catheters), balloons, filters (*e.g*., vena cava filters and mesh filters for distil protection devices), embolization devices including cerebral aneurysm filler coils (including Guglielmi detachable coils and metal coils), drug depots that are adapted for placement in an artery for treatment of the portion of the artery distal to the device, valves including heart valves and vascular valves, and expandable catheters, in particular the Cutting® balloon from Boston Scientific.

Such radially expandable devices typically comprise at least one radially expandable member (e.g., a balloon, stent element, Nitinol mesh or spiral, etc.). Disposed over a surface of the radially expandable member is at least one layer that comprises drug-containing particles.

Drug-containing particles for the invention may contain a wide range of therapeutic agents, as described below. Thus, while antirestenotic agents are typically exemplified herein, the invention is clearly not so-limited.

In various embodiments, the drug-containing particles are hydrophilic particles. Such particles may be hydrophilic because the drug within the particles is hydrophilic. Such particles may be hydrophilic because the particles contain one or more hydrophilic substances in addition to the drug (e.g., admixed with the drug, encapsulating the drug, etc.).

In various embodiments, the drug-containing particles have a net surface charge, for example, because the drug within the particles is charged or because the particles contain one or more charged substances in addition to the drug (e.g., admixed with the drug, encapsulating the drug, etc.). With regard to cellular uptake by living cells (e.g., by pinocytosis, endocytosis, phagocytosis, etc.), charged colloidal particles are ingested faster than uncharged ones, and positively charged particles are found to be ingested faster than negatively charged ones. See, e.g., P.R. Gil et al., NanoToday, 4(3-4), Jun-Aug 2008, 12-21.

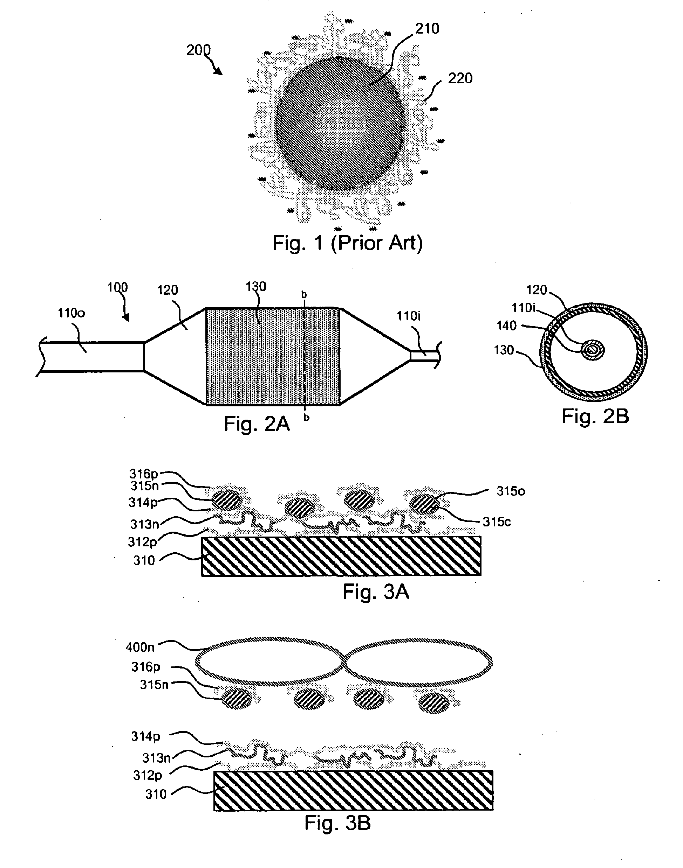

A specific example of a drug-containing particle is Abraxane® in which paclitaxel is complexed with albumin to form stable particles of approximately 130 nm diameter. Abraxane® has been approved by the FDA for the treatment of metastatic breast cancer. Each 50 mL vial of Abraxane® contains approximately 100 mg of paclitaxel and approximately 900 mg of human albumin as a sterile, lyophilized cake. Each vial can be reconstituted with 20 mL of 0.9% Sodium Chloride Injection, USP to produce a suspension containing 5 mg/mL of albumin-bound particles. Without being bound to theory, the suspension is stable at high concentrations due to the net negative charge of the particle (as evidenced by a negative zeta potential), which is understood to be imparted by the presence of the albumin at the particle surface. Such a particle is shown schematically in Fig. 1, which shows a particle 200 having a paclitaxel containing core 210 and an albumin-rich surface 220 having a net negative charge. For further information, see, e.g., Abraxane®, Abraxis BioScience, Inc., ODAC Briefing Package, Oncologic Drugs Advisory Committee Meeting, September 7, 2006. See also US 6,506,405 to Desai et al.

In the above example, drug-containing particles are formed using albumen, which is a polyanion. Other examples of drug-containing particles include those formed using polycations. In either case, the polyanion or polycation may be derivitized with hydrophobic groups, if desired, for example, to influence the physical characteristics of the particles, such as size, hydrophobic core regions, and stability.

J.-H. Kim, Journal of Controlled Release 111 (2006) 228-234, describe self-assembled nanoparticles based on the hydrophobic modification of glycol chitosan as a carrier for paclitaxel. Glycol chitosan is a positively charged polysaccharide which is watersoluble at pH 7.4 (see, e.g., S, Sakai et al., Journal of microencapsulation, 17(6), 2000, 691-699). Hydrophobically modified conjugates of glycol chitosan were prepared by chemically linking 5β-cholanic acid (a hydrophobic acid) to glycol chitosan chains using 1-ethyl-3-(3-dimethylaminopropyl)-carbodiimide chemistry, thereby providing glycol chitosan with hydrophobic pendant groups (i.e., 5β-cholanic acid residues). In phosphate-buffered saline (PBS; pH 7.4), the synthesized conjugates formed stable nano-sized particles, which had hydrophobic cores that were suitable to encapsulate hydrophobic peptides and drugs. Paclitaxel was loaded into the nanoparticles up to 10 wt.% using a dialysis method, providing paclitaxel-loaded nanoparticles of about 400 nm in diameter.

As noted above, expandable medical devices are provided herein which comprise at least one layer that comprises drug-containing particles. Expandable catheters are exemplified herein, but the invention is clearly not so-limited.

One example of such a device is shown in Fig. 2A, which schematically illustrates a balloon catheter 100 comprising an inner tubular member 110i (which may provide a guidewire lumen), an outer tubular member 110o (which may provide an inflation lumen), a balloon 120 and a layer that comprises drug-containing particles 130. Fig. 2B is a cross-section of Fig. 2A taken along line b---b and shows the inner tubular member 110i (within which is disposed a guidewire 140), the balloon 120 and the layer that comprises drug-containing particles 130. Examples of balloon materials include relatively non-complaint materials such as polyamides, for instance, polyamide homopolymers and copolymers and composite materials in which a matrix polymer material, such as polyamide, is combined with a fiber network (e.g., Kevlar® an aramid fiber made by Dupont or Dyneema®, a superstrong polyethylene fiber made by DSM Geleen, the Netherlands). Specific examples of polyamides include nylons, such as nylon 6, nylon 4/6, nylon 6/6, nylon 6/10, nylon 6/12, nylon 11 and nylon 12 and poly(ether-co-amide) copolymers, for instance, polyether-polyamide block copolymer such as poly(tetramethylene oxide)-*b*-polyamide-12) block copolymer, available from Elf Atochem as PEBAX. Examples of balloon materials also include relatively complaint materials such as silicone, polyurethane or compliant grades of PEBAX having a larger percentage of polyether, for example PEBAX 63D. Examples of materials for the inner and outer tubular members 110i and 110o include polyamide, polyethylene (HDPE) and polytetrafluoroethylene (PTFE), among others.

Among other possibilities, a catheter 100 like that of Figs. 2A-2B may be used to deliver an antirestenotic drug concurrently with the delivery of a stent (e.g., to prevent restenosis) or such a catheter 100 may be used to deliver an antirestenotic drug subsequent to the delivery of a stent (e.g., to treat in-stent restenosis).

In certain embodiments, the drug-containing particles are provided on the expandable device by combining the particles with at least one binder material. Preferably, the binder material is selected such that a hard, brittle layer is formed. As defined herein, by a "hard, brittle" layer is meant a layer of material which forms cracks and delaminates from an underlying radially expandable member material upon on full deployment. For example, a fully coated folded balloon may be fully deployed by inflation to 18 atm. at 37C for 60 seconds within a saline environment. The surface of the coated layer is inspected by microscope after the expansion procedure and the coated (i.e., non-delaminated) surface area is measured. When the coated surface area is less than 60%, the coating as such is defined as a "hard, brittle" coating. Note that in the case of a balloon material, this procedure involves inflation from a folded state to a fully deployed round structure. Furthermore, when the pressure in a balloon material is raised from 6 atm. up to 18 atm., even balloon materials defined as "non-compliant" expand in diameter (typically by 10% or more) causing sufficient stress at the interface of the layer. The delamination of the top-coating is therefore caused by a complex combination of geometrical transformation (folded to round) and significant stress formation at the interface. Without wishing to be bound by theory, it is shown that a layer of this type is able to break into fragments upon device expansion, thereby increasing the rate of drug release from the device. It is believed that such brittle fragments may penetrate the surrounding tissue (e.g., vessel wall) upon device expansion, thereby enhancing drug delivery to the surrounding tissue.

Examples of binder materials which may be combined with drug-containing particles to form hard, brittle drug-containing layers include saccharides, for instance, mono-, di- and poly-saccharides, including simple sugars, saccharide oligomers (i.e., compounds containing from 3 to 10 saccharide moieties), and polysaccharides (i.e., compounds containing 11 or more saccharide moieties). In certain embodiments, particular matrix polysaccharides termed glycosaminoglycans (GACs) are employed as they have a beneficial effect of promoting endothelial cell growth while having an inhibitory growth effect on smooth muscle cells. Examples of polysaccharides include chondroitin A, chondroitin sulfate B, chondroitin sulfate C, dextran sulfate, chitosan and combinations of chitosan with the above mentioned polysaccharides. See, e.g., Janeen M. Chupa et al., Biomaterials 21 (2000) 2315-2322. These materials will be especially beneficial in these cases where the expandable device will be in longer contact with the vessel wall, such as grafts, filters or coils. Another example of a polysaccharide is heparin. Standard heparin preparations have molecular weights in the range of 1.35×10⁴ to 1.5×10⁴, although other higher and lower molecular weight preparations may be employed.

Examples of binder materials further include iodine-containing compounds and substituted aromatic compounds, including iodine-substituted aromatic compounds. Examples of binder material include non-ionic Roentgen contrast media. Specific examples include monomeric commercially available contrast agents such as iopromide, iopamidol. iohexol, iopentol or ioversol, which are represented by the general formula, 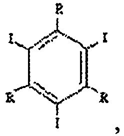 and dimeric commercially available contrast agents such as iotrolan and iodixanol, which are represented by the general formula, 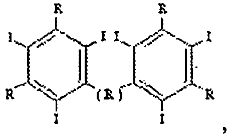 where each R in the preceding equations independently represents an organic moiety. For example, the following structure, 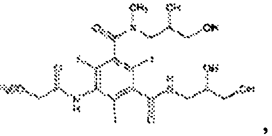 is that of iopromide. Although modem contrast media molecules are more hydrophilic due to longer side chains shielding the highly hydrophobic Iodine atom, as the main purpose for the contrast media molecules in this application is to create a hard, brittle coating layer, in some embodiments, one could employ chemical formulations that are similar to the contrast media, reducing or fully replacing one or more of the iodine atoms (up to all) by other functional groups, such as bromine, chlorine, or even carboxylic acid groups. One can also use different classes of matrix materials such as acetylsalicylic acid (a substituted aromatic molecule), among many others.

In certain specific embodiments, one of the above binder materials (e.g., substituted aromatic compounds, including monomeric and dimeric contrast agents, for instance, acetylsalicylic acid or iopromide) is combined with antirestenotic-drug-containing particles, for instance, paclitaxel-containing particles (e.g., Abraxane® particles), to form a hard, brittle drug-containing layer on the surface of a balloon. Such a strategy represents a novel approach in which drug particles are bound to the balloon surface, rather than simply mixing the drug with the binder.

For instance, Abraxane ® particles and the binder material may be mixed in solution (see, e.g., Example 3 below) before application to the balloon surface, for instance, by means of spraying or inkjet-printing. Multiple layers can be applied by applying a drying step in between.

Furthermore; Abraxane® is in the form of negatively charged 130 nm diameter particles. As indicated above, charged colloidal particles are reported to be ingested faster than uncharged ones, with ingestion of positively charged particles being enhanced relative negatively charged ones. P.R. Gil et al., NanoToday, 4(3-4), Jun-Aug 2008, 12-21. Such particles are also not prone to being transported further into the vessel and forming a blockage (e.g., due to their small size, the fact that they are like charged and thus repel each other, etc.).

In other embodiments of the invention, drug-containing particles are held on the device by electrostatic forces. In certain embodiments, an expandable device is provided which comprises at least one radially expandable member (e.g., a balloon, etc.) and a layer of drug-containing particles that are electrostatically attached to the surface of the radially expandable member.

For example, an expandable device may be provided with at least one radially expandable member (e.g., a balloon, etc.) whose surface has a first net charge, and a layer of drug-containing particles (whose surfaces have a second net charge that is opposite in sign to the first net charge) may be deposited onto the surface where the particles are held by electrostatic attractive forces.

In certain embodiments, the surface of the radially expandable member (e.g., balloon) has an inherent net charge. For example, a net surface charge may arise inherently from the chemical nature of the material from which the expandable member is formed.

To the extent that the expandable member does not have an inherent net surface charge, a surface charge may nonetheless be provided. For example, various materials, including metallic and polymeric materials, may be chemically treated with a variety of reagents, including reducing agents and oxidizing agents (e.g., sulfur trioxide for sulfonate formation), which modify their surfaces so as to provide them with charged groups, such as amino, phosphate, sulfate, sulfonate, phosphonates and carboxylate groups, among many others. A substrate can be provided with a charge by covalently coupling with species having functional groups with a positive charge (e.g., amine, imine or other basic groups) or a negative charge (e.g., carboxylic, phosphonic, phosphoric, sulfuric, sulfonic, or other acid groups) using methods well known in the art. Further information on covalent coupling may be found, for example, in Pub. No. US 2005/0002865.

Other techniques for providing surface charge include techniques whereby a surface region is treated with a reactive plasma. Surface modification is obtained by exposing a surface to a partially ionized gas (i.e., to a plasma). Because the plasma phase consists of a wide spectrum of reactive species (electrons, ions, etc.) these techniques have been used widely for functionalization of surfaces, including polymeric surfaces among others. Examples include glow discharge techniques (which are conducted at reduced pressure) and coronal discharge techniques (which are conducted at atmospheric pressure), with the former preferred in some cases, because the shape of the object to be treated is of minor importance during glow discharge processes. Lasers may also be used to create a localized plasma in the vicinity of the laser beam (e.g., just above the focal point of the beam). When gases like carbon monoxide (CO), carbon dioxide (CO₂), or oxygen (O₂) are used, functionalization with -COOH groups (which donate protons to form anionic groups) is commonly formed. When gases like ammonia, a propyl amine, or N₂/H₂ are employed, -NH₂ groups (which accept protons to form cationic groups) are commonly formed. Functional-group-containing surfaces may also be obtained using plasma polymerization processes in which "monomers" are employed that contain functional groups. Allylamine (which produces -NH₂ groups) and acrylic acid (which produces -COOH groups) have been used for this purpose. By using a second feed gas (generally a non-polymerizable gas) in combination with the unsaturated monomer, it is possible to incorporate this second species in the plasma deposited layer. Examples of gas pairs include allylamine/NH₃ (which leads to enhanced production of -NH₂ groups) and acrylic acid/CO₂ (which leads to enhanced production of -COOH groups). Further information on plasma processing may be found, for example, in "Functionalization of Polymer Surfaces," Europlasma Technical Paper, 05/08/04 and in Pub. No. US 2003/0236323.

In other embodiments, a surface charge is provided on a substrate simply by adsorbing polycations or polyanions to the surface of the substrate as a first charged layer. Polyethyleneimine (PEI) is commonly used for this purpose, as it strongly promotes adhesion to a variety of substrates. Further information can be found in Pub. No. US 2007/0154513 to Atanasoska et al. In certain embodiments, a single charged layer (e.g., a layer of PEI) is deposited prior to deposition of a layer of drug-containing particles of opposite net charge.

In certain other embodiments, multiple charged layers are deposited prior to deposition of the drug-containing particles. In this regard, it is known that coatings can be formed on substrates based on electrostatic self-assembly of charged materials. In these processes, for example, a first charged material having a first net charge is typically deposited from a first solution or dispersion onto an underlying charged substrate, followed by deposition of a second charged material (which has a second net charge that is opposite in sign to the net charge of the first material) from a second solution or dispersion, and so forth. The net charge on the outer surface is reversed upon deposition of each sequential layer. Commonly, 2 to 5 to 10 to 25 to 50 to 100 to 200 or more layers may be applied in this technique, depending on the desired thickness of the coating. Examples of charged materials include charged large molecules (e.g., charged polymers) and charged particles, among others. For further information concerning layer-by-layer electrostatic self-assembly methods, see, e.g., US 2005/0208100 to Weber et al., and WO/2005/115496 to Chen et al.

Thus, regardless of the method by which a given substrate is provided with a surface charge, once a sufficient net positive or negative surface charge is provided (e.g., via chemical conversion of the surface, adsorption/binding of charged species onto the surface, etc.), the substrate can be readily coated with one or more layers of materials of alternating net charge, including various charged polymers and charged particles.

"Charged polymers" are polymers having multiple charged groups. (Such polymers are also referred to herein as "polyelectrolytes.") Charged polymers thus include a wide range of species, including polycations and their precursors (e.g., polybases, polysalts, etc.), polyanions and their precursors (e.g., polyacids, polysalts, etc.), ionomers (charged polymers in which a small but significant proportion of the constitutional units carry charges), and so forth. Typically, the number of charged groups is so large that the polymers are soluble in polar solvents (particularly water) when in ionically dissociated form (also called polyions). Some charged polymers have both anionic and cationic groups (e.g., peptides, proteins, etc.) and may have a negative net charge (e.g., because the anionic groups contribute more charge than the cationic groups), a positive net charge (e.g., because the cationic groups contribute more charge than the anionic groups), or may have a neutral net charge (e.g., because the cationic groups and anionic groups contribute equal charge). In this regard, the net charge of a particular charged polymer may change with the pH of its surrounding environment. Charged polymers containing both cationic and anionic groups may be categorized herein as either polycations or polyanions, depending on which groups predominate at neutral pH. (Clearly, charged polymers with only anionic groups have a negative net charge, while charged polymers having only cationic groups have a positively net charge.)

Specific examples of polycations include, for instance, polyamines, including poly(amino methacrylates) including poly(dialkylaminoalkyl methacrylates) such as poly(dimethylaminoethyl methacrylate) and poly(diethylaminoethyl methacrylate), polyvinylamines, polyvinylpyridines including quaternary polyvinylpyridines such as poly(N-ethyl-4-vinylpyridine), poly(vinylbenzyltrimethylamines), polyallylamines such as poly(allylamine hydrochloride) (PAH) and poly(diallyldialklylamines) such as poly(diallyldimethylammonium chloride), polyamidoamines, polyimines including polyalkyleneimines such as poly(ethylene imine) (PEI), poly(propylene imine) and ethoxylated poly(ethylene imines), polycationic peptides and proteins, including histone peptides and homopolymer and copolymers containing basic amino acids such as lysine, arginine, ornithine and combinations thereof, gelatin, protamine and protamine sulfate, spermine, spermidine, hexadimethrene bromide (polybrene), and polycationic polysaccharides such as cationic starch and chitosan, as well as copolymers, salts, derivatives and combinations of the preceding, among various others.

Specific examples of polyanions include, for instance, polysulfonates such as polyvinylsulfonates, poly(styrenesulfonates) such as poly(sodium styrenesulfonate) (PSS), sulfonated poly(tetrafluoroethylene), sulfonated polymers such as those described in U.S. Patent No. 5,840,387, including sulfonated styrene-ethylene/butylene-styrene triblock copolymers, sulfonated styrenic homopolymers and copolymers such as a sulfonated versions of the polystyrene-polyolefin copolymers described in U.S. Patent No. 6,545,097 to Pinchuk et al., which polymers may be sulfonated, for example, using the processes described in U.S. Patent No. 5,840,387 and U.S. Pat. No. 5,468,574, as well as sulfonated versions of various other homopolymers and copolymers, polysulfates such as polyvinylsulfates, sulfated and non-sulfated glycosaminoglycans as well as certain proteoglycans, for example, heparin, heparin sulfate, chondroitin sulfate, keratan sulfate, dermatan sulfate, polycarboxylates such as acrylic acid polymers and salts thereof (e.g., ammonium, potassium, sodium, etc.), for instance, those available from Atofina and Polysciences Inc., methacrylic acid polymers and salts thereof (e.g., EUDRAGIT, a methacrylic acid and ethyl acrylate copolymer), carboxymethylcellulose, carboxymethylamylose and carboxylic acid derivatives of various other polymers, polyanionic peptides and proteins such as homopolymers and copolymers of acidic amino acids such as glutamic acid, aspartic acid or combinations thereof, homopolymers and copolymers of uronic acids such as mannuronic acid, galatcuronic acid and guluronic acid, and their salts, alginic acid and its salts, hyaluronic acid and its salts, albumin, gelatin, carrageenan, polyphosphates such as phosphoric acid derivatives of various polymers, polyphosphonates such as polyvinylphosphonates, as well as copolymers, salts, derivatives, and combinations of the preceding, among various others.

In accordance with the present invention, an expandable member (e.g., a balloon) may be provided with a first polycationic layer, followed by a first polyanionic layer, followed by a second polycationic layer, followed by a second polyanionic layer, and so forth. Conversely, an expandable member may be provided with a first polyanionic layer, followed by a first polycationic layer, followed by a second polyanionic layer, followed by a second polycationic layer, and so forth. If a layer of drug-containing particles with a positive net surface charge is to be deposited, then the last polyelectrolyte layer to be deposited before the particles will be a polyanionic layer. If a layer of drug-containing particles with a negative net surface charge is to be deposited, then the last polyelectrolyte layer to be deposited before the particles will be a polycationic layer.

Various deposition techniques may be employed, including dipping, spraying, ink-jet printing, rolling, and in general all other wet deposition techniques.

As with other materials, drug-containing particles can be provided with a charge by adsorption/binding of charged polyelectrolyte (e.g., PEI) onto the surface.

Similarly, the net charge on the surface of drug-containing particles may be changed by deposition of a polyelectrolyte layer having a net charge that is opposite to that of the particles. The net charge may be changed, for example, either before or after application of the drug-containing particles to the expandable member.

Turning now to Fig. 3A, a portion of a radially expandable member such as a balloon 310 is schematically illustrated. On the surface of the balloon 310 is a first positively charged polyelectrolyte layer 312p (designated by light gray lines), followed by a first negatively charged polyelectrolyte layer 313n (designated by dark gray lines), followed by a second positively charged polyelectrolyte layer 314p (designated by light gray lines). After deposition of the second positively charged polyelectrolyte layer 314p, a layer of negatively charged particles 315n is deposited. The particles include a core 315c comprising a hydrophobic drug (e.g., paclitaxel) and a negatively charged outer surface 315o. In this embodiment, it is desired to reverse the net surface charge of the particles, so a third positively charged polyelectrolyte layer 316p (designated by light gray lines) is deposited. Note that the third positively charged polyelectrolyte layer 316p will typically preferentially deposit on the particles 315n, which are negatively charged, relative to the underlying polyelectrolyte layer 314p lying between the particles, which is positively charged (although mechanisms other than electrostatic forces may lead to such binding).

Fig. 3B is a schematic illustration of the drug delivery device portion of Fig. 3A after coming into contact with a cell wall 400. Due to the application of the positively charged polyelectrolyte layer 316p, the particles will be electrostatically attracted to negatively charged surfaces such as cell walls of the vasculature (e.g., blood vessel walls), promoting removal of the particles from the device surface as shown.

A wide variety of therapeutic agents can be employed in conjunction with the present invention for the treatment of a variety of diseases and conditions (i.e., the prevention of a disease or condition, the reduction or elimination of symptoms associated with a disease or condition, or the substantial or complete elimination of a disease or condition) in various vertebrate subjects (e.g., humans, pets, livestock, etc.).

Therapeutic agents for use in connection with the present invention include: (a) antithrombotic agents such as heparin, heparin derivatives, urokinase, clopidogrel, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan, eprosartan and telmisartan), and antispasmodic/anticholinergic drugs (e.g., oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.), (z) selective estrogen receptor modulators (SERMs) such as raloxifene, lasofoxifene, arzoxifene, miproxifene, ospemifene, PKS 3741, MF 101 and SR 16234, (aa) PPAR agonists, including PPAR-alpha, gamma and delta agonists, such as rosiglitazone, pioglitazone, netoglitazone, fenofibrate, bexaotene, metaglidasen, rivoglitazone and tesaglitazar, (bb) prostaglandin E agonists, including PGE2 agonists, such as alprostadil or ONO 8815Ly, (cc) thrombin receptor activating peptide (TRAP), (dd) vasopeptidase inhibitors including benazepril, fosinopril, lisinopril, quinapril, ramipril, imidapril, delapril, moexipril and spirapril, (ee) thymosin beta 4, (ff) phospholipids including phosphorylcholine, phosphatidylinositol and phosphatidylcholine, (gg) VLA-4 antagonists and VCAM-1 antagonists.

Preferred non-genetic therapeutic agents include taxanes such as paclitaxel, olimus family drugs such as sirolimus, everolimus, tacrolimus, zotarolimus and biolimus, Epo D, dexamethasone, estradiol, halofuginone, cilostazole, geldanamycin, alagebrium chloride (ALT-711), ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well derivatives of the forgoing, among others.

Numerous therapeutic agents, not necessarily exclusive of those listed above, have been identified as candidates for vascular treatment regimens, for example, as agents targeting restenosis (antirestenotics). Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists such as bosentan, sitaxsentan sodium, atrasentan, endonentan, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, atorvastatin, fluvastatin, simvastatin and cerivastatin, (u) fish oils and omega-3-fatty acids, (v) free-radical scavengers/antioxidants such as probucol, vitamins C and E, ebselen, trans-retinoic acid, SOD (orgotein) and SOD mimics, verteporfin, rostaporfin, AGI 1067, and M 40419, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) matrix metalloprotease (MMP) pathway inhibitors such as marimastat, ilomastat, metastat, batimastat, pentosan polysulfate, rebimastat, incyclinide, apratastat, PG 116800, RO 1130830 or ABT 518, (y) cell motility inhibitors such as cytochalasin B, (z) antiproliferative/antineoplastic agents including antimetabolites such as purine antagonists/analogs (e.g., 6-mercaptopurine and pro-drugs of 6-mercaptopurine such as azathioprine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), olimus family drugs (e.g., sirolimus, everolimus, tacrolimus, zotarolimus, etc.), cerivastatin, flavopiridol and suramin, (aa) matrix deposition/organization pathway inhibitors such as halofuginone or other quinazolinone derivatives, pirfenidone and tranilast, (bb) endothelialization facilitators such as VEGF and RGD peptide, (cc) blood rheology modulators such as pentoxifylline and (dd) glucose crosslink breakers such as alagebrium chloride (ALT-711).

Numerous additional therapeutic agents useful for the practice of the present invention are also disclosed in U.S. Patent No. 5,733,925 to Kunz.

### Example 1

The surface of a PEBAX angioplasty balloon (Apex ®, Boston Scientific, 18 mm long middle section, 3.5 mm diameter when inflated @ 15atm) is pre-treated to improve the adhesion of an initial polyelectrolyte layer. The balloon may be pre-treated, for example, by plasma etching (e.g., 30 seconds in an oxygen plasma with an RF power of 650 watts) or chemical etching (e.g., 10-30 min in a solution of 50% concentrated HCl and 50 % concentrated H₂O₂).

The following solutions are prepared: (a) a cationic poly(ethylene imine) (PEI) solution containing (1mg/ml PEI MW 60,000 branched from Sigma Aldrich ; 0.2M NaCl; pH 5.6; (b) a cationic poly(allylamine hydrochloride) (PAH) solution (1mg/ml; 0.2M NaCl; pH 5.6); (c) an anionic poly(sodium 4-styrenesulfonate) (PSS) solution (1mg/ml; 0.8M NaCl; pH 5.6); and (d) an Abraxane® solution prepared by dissolving/dispersing 100 mg of paclitaxel and approximately 900 mg of human albumin with 20 mL of 0.9% Sodium Chloride Injection, suspension containing 5 mg/mL of albumin-bound particles.

An initial layer of poly(ethylene imine) (PEI) is created on the balloon by dipping the balloon into the PEI solution at room temperature for 30 seconds. The balloon is inflated during dipping to 8 atm. After that 6 additional layers of PSS and PAH are deposited in alternation by dipping in the PSS and PAH solutions. Each dip step is performed at room temperature and lasts for 30 seconds. The balloon is washed twice in deionized water between the dipping steps. One could additionally replace the PAH by another cationic polyelectrolyte such as chitosan, poly-1-lysine, protamine, polyethylenimine, poly-1-arginine or poly (diallyl-dimethyl-ammoniumchloride) (PDADMAC), among others, and/or one could replace the PSS with another anionic polyelectrolyte such as poly-1-glutamic acid, poly-1-aspartic acid, hyaluronic acid, heparin, Eudragit L, polyacrylic acid or polymethacrylic acid, among others. Finally, the balloon is dipped in the Abraxane® solution for 30 seconds to obtain a homogeneous layer of Abraxane® particles onto the balloon surface. The balloon is then dried in an oven at 80°C for four hours in order to obtain a dry coating.

If desired, after the application of the layer of Abraxane® particles, the balloon may be dipped again in a solution of a strong cationic polyelectrolyte such as PDADMAC (1mg/ml; 0.2M NaCl; pH 5.6). PDADMAC is a strong cationic polyelectrolyte and will as such form a strong connection with the negatively charged Albumin of Abraxane® particles. The resulting positively charged particles will adhere strongly to negatively charged cell membranes such as those of the vasculature (e.g., blood vessel wall), promoting removal of the particles from the balloon surface, which particles are held in place by weak electrostatic bonds.

### Example 2

As in Example 1, the balloon is pretreated and covered with an intial layer of PEI, followed by 6 alternating layers of PSS\PAH and a top layer of an anionic PSS layer. 1 ml of the solution of Abraxane® particles as described in Example 1 is slowly mixed with 2 ml of chitosan (1mg/ml; 0.2M NaCl pH 5.6). The Abraxane® particles as such will be covered with a positively charged chitosan layer. Finally, the balloon surface is dipped in this Abraxane®-chitosan solution and dried similar to Example 1.

### Example 3

The surface of a PEBAX angioplasty balloon is pretreated as described above in Example 1. 10 ml of a solution containing Abraxane® (1 mg/ml) in water is mixed with 16.04 ml of ULTRAVIST 300 (0.6234 g Iopromid /ml). This solution is sprayed on the balloon surface, while the balloon is inflated to a pressure of only 3 atm. This is important as to increase the stress in the hard, brittle coating layer upon expansion in the body and to promote the breakage of the coating necessary to deliver the pharmaceutical substance to the body tissue. In between coating steps, the balloon is blown dry using heated air @ 80C. The spraying process is repeated until a dried coating layer of approximately 20 micrometer thickness is obtained. The balloon is then dried in an oven at 80°C for four hours in order to obtain a fully dry coating.

### Example 4

Similar to Example 2, a stent (Liberté stent, Boston Scientific) is covered with an intial layer of PEI, followed by 6 alternating layers of PSS\PAH and a top layer of an anionic PSS layer. A solution is made of chitosan and Abraxane (with an albumin outer layer), whereby in this case 1 ml of the solution of Abraxane® particles as described in Example 1 is slowly mixed with 10 ml of Chitosan (1mg/ml; 0.2M NaCl pH 5.6). This solution is sprayed @ 50°C on top of the stent surface at a layer thickness of 3 micrometer.

### Example 5

In this example, a hydrogel coating with a drug load consisting of paclitaxel-loaded modified-glycol-chitosan nanoparticles is created. The glycol chitosan does not form a complex with the hydrogel solution and is freely released in response to compression of the polymer. All materials can be obtained from Sigma-Aldrich.

As a first step a hydrogel coating on an angioplasty balloon (Apex ®, Boston Scientific,18 mm long middle section) is formed as follows. The balloon is coated with a solution of 4,4' diphenylmethane diisocyanate (MDI) in methylethylketone for 30 minutes. After drying in an air oven at 85°C for 30 minutes, the balloon is dipped in a 5 % solution of poly(acrylic acid) homopolymer having a molecular weight of about 3,000,000 in dimethylformamide (DMF) and tertiarybutyl alcohol. The balloon is oven dried for 8 hours at 50°C. The polyisocyanate solution is at a concentration of about 5 % by weight. The polyacrylic acid is at a concentration of about 5 % by weight. The targeted poly(carboxylic acid) to polyisocyanate molar ratio is generally about 1:1 and within in a range of 0.5% to 10% by weight.

As a second step, paclitaxel-containing modified-glycol-chitosan nanoparticles are prepared following the procedure as described in S. Kwon et al., "Physicochemical characteristics of self-assembled nanoparticles based on glycol Chitosan bearing 5β Cholanic acid," Langmuir, 19 (2003) 10188-10193 and Jong-Ho Kim et al., "Hydrophobically modified glycol chitosan nanoparticles as carriers for paclitaxel," Journal of Controlled Release, 111 (2006) 228-234)"). Specifically, glycol chitosan (500 mg; 2 µM) is hydrophobically modified with cholanic acid (0.0115-0.345 mol/mol glucosamine) in 1:1 (v/v) methanol/water for 24 h at room temperature. To activate the carboxylic acid groups of cholanic acid, equal amounts of 1-ethyl-3-(3-dimethylaminopropyl)- carbodiimide hydrochloride and N-hydroxysuccinimide are added. The reaction mixture is dialyzed for 3 days against 1:4 (v /v) water/methanol and then lyophilized. The as-made hydrophobically modified glycol chitosan (HGC) (10 mg) are dissolved in 2 ml of 1:1 (v /v) water/methanol, and 1 mg paclitaxel (PTX) in 1 ml of methanol is added to the HGC solution. The solution is vigorously stirred for 12 h at room temperature and then dialyzed against water using a membrane with a molecular weight cutoff of 12,000-14,000 (Spectrapor, Rancho Dominquez, CA). The supernatant is filtered through a 0.8-µm membrane filter and lyophilized.

The as-produced PTX-HGC nanoparticles (2 mg/ml) are dispersed in water and 20 ml of this solution is made after which the hydrogel coated balloon is dipped in this solution for 1 minute at room temperature. The balloon is oven dried for 8 hours at 50°C.

## Claims

1. A drug delivery device for delivery of a drug to a body lumen, said drug delivery device comprising a radially expandable member and a hard, brittle layer disposed over the radially expandable member that forms cracks and delaminates from the member upon expansion of the member, said hard, brittle layer comprising a binding material comprising one or more materials selected from monosaccharides, disaccharides, oligosaccharides and polysaccharides and particles that comprise an antirestenotic drug.

2. The drug delivery device of claim 1, wherein said particles comprise a core that comprises a hydrophobic drug and a surface region that comprises a hydrophilic species.

3. The drug delivery device of claim 2, wherein said hydrophilic species is a polyelectrolyte.

4. The drug delivery device of claim 1, wherein said particles comprise paclitaxel and albumin.

5. The drug delivery device of claim 1, wherein said particles comprise an antirestenotic drug and chitosan.

6. The drug delivery device of claim 5, wherein said chitosan is a glycol chitosan comprising hydrophobic pendant groups.

## Patentansprüche

1. Medikamentenzufuhr-(Drug Delivery-)Vorrichtung zur Zufuhr eines Medikaments in ein Körperlumen, wobei die Medikamentenzufuhr-(Drug Delivery-)Vorrichtung ein radial aufweitbares Teil und eine über dem radial aufweitbaren Teil angeordnete harte spröde Schicht aufweist, die bei der Aufweitung des Teils Risse bildet und sich von dem Teil löst, wobei die harte spröde Schicht ein Bindematerial, das eines oder mehrere aus Monosacchariden, Disacchariden, Oligosacchariden und Polysacchariden ausgewählte Materialen aufweist, und Partikel aufweist, die ein Anti-Restenose-Medikament enthalten.

2. Medikamentenzufuhr-(Drug Delivery-)Vorrichtung nach Anspruch 1, wobei die Partikel einen Kern mit einem hydrophoben Medikament und einen Oberflächenbereich mit einer hydrophilen Substanz aufweisen.

3. Medikamentenzufuhr-(Drug Delivery-)Vorrichtung nach Anspruch 2, wobei die hydrophile Substanz ein Polyelektrolyt ist.

4. Medikamentenzufuhr-(Drug Delivery-)Vorrichtung nach Anspruch 1, wobei die Partikel Paclitaxel und Albumin aufweisen.

5. Medikamentenzufuhr-(Drug Delivery-)Vorrichtung nach Anspruch 1, wobei die Partikel ein Anti-Restenose-Medikament und Chitosan aufweisen.

6. Medikamentenzufuhr-(Drug Delivery-)Vorrichtung nach Anspruch 5, wobei das Chitosan ein Glykol-Chitosan ist, das angehängte hydrophobe Gruppen aufweist.

## Revendications

1. Dispositif d'administration d'un médicament dans une lumière corporelle, ledit dispositif d'administration d'un médicament comprenant un élément radialement expansible et une couche dure friable disposée sur l'élément radialement expansible, qui forme des fissures et se détache de l'élément lors de l'expansion dudit élément, ladite couche dure friable comportant une matière de liaison composée d'une ou de plusieurs substances sélectionnées parmi les monosaccharides, les disaccharides, les oligosaccharides et les polysaccharides, et des particules comprenant un agent anti-resténotique.

2. Dispositif d'administration d'un médicament selon la revendication 1, où les particules comprennent un noyau contenant un agent hydrophobe et une zone de surface contenant une espèce hydrophile.

3. Dispositif d'administration d'un médicament selon la revendication 2, où l'espèce hydrophile est un polyélectrolyte.

4. Dispositif d'administration d'un médicament selon la revendication 1, où les particules contiennent du paclitaxel et de l'albumine.

5. Dispositif d'administration d'un médicament selon la revendication 1, où les particules contiennent un agent anti-resténotique et du chitosane.

6. Dispositif d'administration d'un médicament selon la revendication 5, où le chitosane est un glycol-chitosane contenant des groupes pendants hydrophobes.
